(19) Europäisches Patentamt

European Patent Office

Office européen des brevets

(11) **EP 0 998 438 B1**

(12) **EUROPÄISCHE PATENTSCHRIFT**

(45) Veröffentlichungstag und Bekanntmachung des Hinweises auf die Patenterteilung:
**24.07.2002 Patentblatt 2002/30**

(21) Anmeldenummer: **98934999.8**

(22) Anmeldetag: **26.06.1998**

(51) Int Cl.$^7$: **C07C 17/04**, C07C 19/16

(86) Internationale Anmeldenummer:
**PCT/EP98/03909**

(87) Internationale Veröffentlichungsnummer:
**WO 99/01412 (14.01.1999 Gazette 1999/02)**

(54) **KONTINUIERLICHES VERFAHREN ZUR HERSTELLUNG VON PENTAFLUORETHYLIODID**

CONTINUOUS METHOD FOR PRODUCING PENTAFLUOROETHYL IODIDE

PROCEDE CONTINU DE PRODUCTION D'IODURE DE PENTAFLUOROETHYLE

(84) Benannte Vertragsstaaten:
**BE CH DE FR GB IT LI NL**

(30) Priorität: **04.07.1997 DE 19728560**

(43) Veröffentlichungstag der Anmeldung:
**10.05.2000 Patentblatt 2000/19**

(73) Patentinhaber: **Clariant GmbH**
**65929 Frankfurt am Main (DE)**

(72) Erfinder:
- **RICHTER, Hans-Bodo**
  **D-84489 Burghausen (DE)**
- **PAUL, Norbert**
  **D-84503 Altötting (DE)**
- **HUBER, Rudolf**
  **D-84518 Garching (DE)**

(56) Entgegenhaltungen:
**US-A- 3 006 973**    **US-A- 3 406 214**

EP 0 998 438 B1

**Beschreibung**

Pentafluorethyliodid ist ein wertvolles Synthon zur Einführung der Pentafluorethylgruppe in organische Verbindungen, die beispielsweise herbizide Wirkung aufweisen. Die größte Bedeutung hat Pentafluorethyliodid jedoch als Telogen in der sogenannten Telomerisation mit Tetrafluorethylen und/oder Hexafluorpropen erlangt. Die hierbei entstehenden Perfluoralkyliodide sind wichtige Edukte für zahlreiche Synthesen, die Wirkstoffe mit stark ausgeprägten hydrophoben und oleophoben Eigenschaften liefern.

Pentafluorethyliodid wird vorwiegend gemäß folgender Gleichung (I) aus Tetrafluorethylen, Iodpentafluorid und Iod hergestellt:

$$5CF_2=CF_2 + IF_5 + 2I_2 \rightarrow 5CF_3CF_2I \qquad (I)$$

Ein entsprechender Prozeß ist beispielsweise in US-A-3 406 214 beschrieben. Hierin wurde eine Mischung aus Iodpentafluorid und 10 Gew.-% Iod bei 60 bis 80 °C mit gasförmigen Tetrafluorethylen umgesetzt. Es wurde ein Umsatz von 40 Gew.-% und eine Ausbeute von 90 Gew.-% an Pentafluorethyliodid erzielt.

Es hat nicht an Versuchen gefehlt, das Herstellverfahren gemäß obiger Gleichung (I) mit Katalysatoren zu beschleunigen, insbesondere mit Katalysatoren aus der Gruppe der Lewis-sauren Metallverbindungen, beispielsweise $TiCl_4$, $ZrCl_4$ oder $VF_5$ (siehe DE-C-20 33 755). Nachteilig wirkt sich jedoch bei diesem und ähnlichen Verfahren die erhöhte Korrosionsrate an den üblicherweise verwendeten Edelstahl-Apparaten aus.

Angesichts des Gefahrenpotentials, das insbesondere vom extrem reaktiven und toxischen Iodpentafluorid ausgeht, muß eine Herstellanlage für Pentafluorethyliodid nach dem Verfahren gemäß Gleichung (I) besonderen Anforderungen bezüglich Dichtheit genügen und möglichst wenig bewegte Teile aufweisen. Eine Zugabe des Iods in verflüssigter Form (durch Aufschmelzen unter Druck) erwies sich wegen der Korrosivität gegen metallische Werkstoffe als nicht empfehlenswert; selbst Nickel-Basislegierungen sind bei der Lagerung von flüssigem Iod nicht vollkommen beständig. Darüber hinaus erwies sich eine exakte Dosierung des unter Druck stehenden, flüssigen, circa 125 °C heißen Iods zu dem im Reaktor befindlichen circa 90 °C heißen Iodpentafluorid als problematisch.

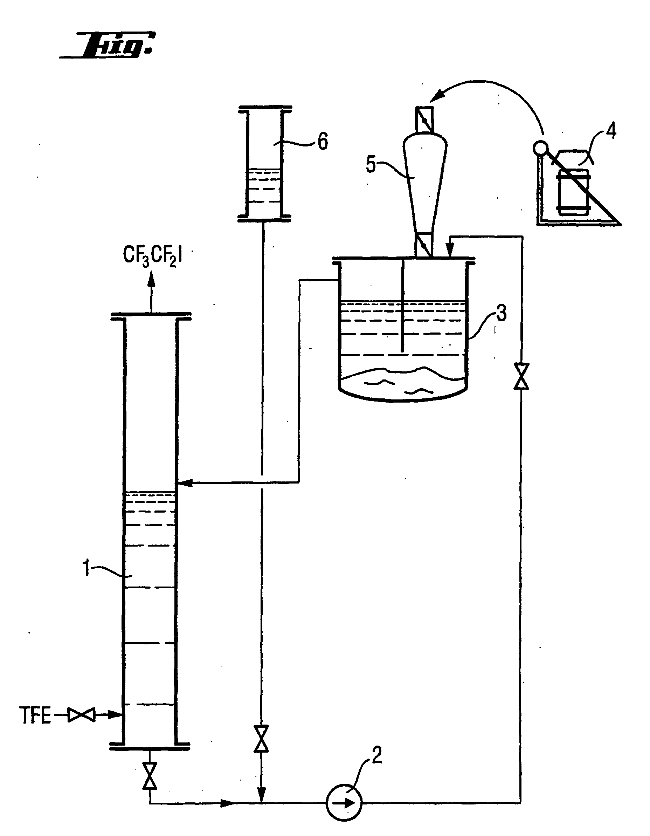

Es bestand somit ein großer Bedarf für ein sicheres und einfaches Verfahren zur Herstellung von Pentafluorethyliodid gemäß Gleichung (I). Die erfinderische Aufgabe wird im wesentlichen durch ein kontinuierliches Lösen von festem Iod in Iodpentafluorid gelöst. Das Fließschema des Verfahrens geht aus der Figur hervor. Im einzelnen besteht das Verfahren aus folgenden Schritten:

Ein vertikaler Blasensäulenreaktor 1, der mit einer Füllstandsregelung ausgerüstet ist, wird mit $IF_5$ befüllt und auf 85 bis 95 °C, vorzugsweise 90 °C, aufgeheizt. Aus einem Iod-Transportbehälter wird mit einer Hebe- und Kippvorrichtung 4 eine definierte Menge von kristallinem Iod in die Befüllschleuse 5 eingebracht und von dort aus chargenweise in das mit $IF_5$ befüllte Iod-Lösegefäß 3 gegeben. In das Iod-Lösegefäß 3 wird kontinuierlich "abgemagertes" $IF_5$ aus dem Blasensäulenreaktor 1 mit der Kreislaufpumpe 2 gefördert. Das Iod-Lösegefäß 3 ist so gestaltet, daß die Befüllung mit festem Iod und die Einleitung des "abgemagerten" $IF_5$ durch eine Beruhigungszone vom Überlauf des mit Iod aufkonzentrierten $IF_5$ getrennt ist. Das mit Iod bis zum Lösungsgleichgewicht aufkonzentrierte $IF_5$ läßt man kontinuierlich über einen Überlauf in den Reaktor 1 fließen, während man gleichzeitig am Fuß des Reaktors 1 Tetrafluorethylen in dem Maße einleitet, wie es durch die Reaktion verbraucht wird. Reines Pentafluorethyliodid entweicht am Kopf des Reaktors 1, wird in einem gekühlten Kondensator verflüssigt und in einem Vorratstank gesammelt. Das bei der Reaktion verbrauchte $IF_5$ wird über eine Füllstandsregelung im Blasensäulenreaktor 1 aus dem Vorratsbehälter 6 über die Pumpe 2 in den $IF_5$-Kreislauf nachdosiert. Die im Lösegefäß 3 befindliche Menge an festem Iod wird durch eine radioaktive Füllstandsmessung ermittelt. Die Löslichkeit von Iod in Iodpentafluorid beträgt bei 20 °C 5,2 Gew.-% und bei 85 bis 95 °C 9 bis 10 Gew.-%.

Das erfindungsgemäße Verfahren weist folgende Vorteile auf:

a) Es wird kein flüssiges Iod verwendet. Damit entfallen die großen Probleme, die sich aus dem Umgang mit Flüssigiod sowohl verfahrenstechnisch als auch in Hinsicht auf Arbeitssicherheit ergeben.

b) Apparate, Rohrleitungen und Armaturen können aus üblichen Chrom-Nickel-Stählen, beispielsweise Stahl 1.4571, angefertigt werden. Dies bedeutet deutlich niedrigere Investitionskosten im Vergleich zu Flüssigiod-Verfahren.

c) Die Ausbeute an Pentafluorethyliodid beträgt im Dauerbetrieb 97 bis 98 % der theoretischen Ausbeute, bezogen auf $IF_5$. Dies entspricht der besten Ausbeute, die unter Verwendung von Lewis-Säuren wie $SbF_3$ oder $PCl_5$ als Katalysatoren erreicht wurde (siehe JP-A-60/023333). Es liegt auf der Hand, daß ein großer Vorteil des erfindungsgemäßen Verfahrens darin besteht, daß es ohne Lewis-Säuren oder sonstige Katalysatoren durchgeführt wird.

**Patentansprüche**

1. Kontinuierliches Verfahren zur Herstellung von rei-

nem Pentafluorethyliodid aus Iod, Iodpentafluorid und Tetrafluorethylen, **dadurch gekennzeichnet, dass** das Iod in einem abgemagerten Iodpentafluorid gelöst wird, welches vom unteren Ende eines vertikalen Blasenreaktors (1) abgeleitet wird, und die erhaltene Lösung kontinuierlich einem vertikalen Blasensäulenreaktor (1) zugeführt wird, der mit einer Füllstandsregelung ausgerüstet ist, mit Iodpentafluorid befüllt ist und an seinem unteren Ende eine regelbare Zuleitung für Tetrafluorethylen aufweist, wobei die Reaktionszone auf 85 bis 95°C, vorzugsweise 90°C, gehalten wird und das gebildete Pentafluorethyliodid am Kopf des Reaktors (1) gasförmig entweicht, in einem nachgeschalteten gekühlten Kondensator verflüssigt wird und in Vorratsgefäß abgefüllt wird.

**2.** Verfahren nach Anspruch 1, **dadurch gekennzeichnet, daß** kristallines Iod aus geeigneten Vorratsgebinden mittels einer Hebe- und Kippvorrichtung (4) in eine Befüllschleuse (5) gegeben wird, aus der die chargenweise Dosierung des Iods in ein Lösegefäß (3) erfolgt, in dem das Iod in Iodpentafluorid gelöst wird.

**3.** Verfahren nach Anspruch 1 oder 2, **dadurch gekennzeichnet, daß** man gemäß dem Verbrauch kontinuierlich Iodpentafluorid aus einem Vorratsbehälter (6) mit einer Pumpe (2) dem Kreislauf zuführt, der über das Iod-Lösefäß (3) zum Reaktor (1) führt.

## Claims

**1.** A continuous process for the preparation of pure pentafluoroethyl iodide from iodine, iodine pentafluoride and tetrafluoroethylene, which comprises dissolving the iodine in a depleted iodine pentafluoride which is drawn off from the lower end of a vertical bubble reactor (1), and continuously feeding the resulting solution to a vertical bubble column (1), which is equipped with a level regulator, is filled with iodine pentafluoride and at its lower end has a controllable feed line for tetrafluoroethylene, where the reaction zone is maintained at from 85 to 95°C, preferably 90°C, and the pentafluoroethyl iodide formed escapes in gaseous form at the top of the reactor (1), is liquefied in a downstream cooled condenser and is drawn off into a storage vessel.

**2.** The process as claimed in claim 1, wherein crystalline iodine is fed from suitable storage containers by means of a lifting and tilting device (4) to a charge transfer tube (5), from which the batchwise addition of the iodine into a dissolution vessel (3) takes place, where the iodine is dissolved in iodine pentafluoride.

**3.** The process as claimed in claim 1 or 2, wherein iodine pentafluoride is fed continuously at the rate at which it is consumed from a storage container (6) using a pump (2) to the circuit which leads via the iodine dissolution vessel (3) to the reactor (1).

## Revendications

**1.** Procédé continu de production d'iodure de pentafluoroéthyle pur à partir d'iode, de pentafluorure d'iode et de tétrafluoroéthylène, **caractérisé en ce qu'**on dissous l'iode dans un pentafluorure d'iode appauvri, qui est introduit de l'extrémité inférieure d'un réacteur à bulles (1) vertical, et la solution obtenue est chargée en continu dans un réacteur vertical en colonne à bulles (1), qui est équipé d'un régulateur de niveau, est chargée avec du pentafluorure d'iode et présente à son extrémité inférieure une alimentation ajustable pour le tétrafluoroéthylène, ce par quoi la zone de réaction est maintenue de 85 à 95°C, de préférence à 90°C et l'iodure de pentafluoroéthyle formé s'échappe à l'état gazeux en tête du réacteur (1), est liquéfié dans un condensateur refroidi placé en aval et est introduit dans un récipient de stockage.

**2.** Procédé selon la revendication 1, **caractérisé en ce que** l'iode cristallisé est versé d'un fut métallique de stockage approprié au moyen d'un dispositif de levage et de basculement (4) dans une trémie de remplissage (5), de laquelle l'introduction dosée discontinue de l'iode est réalisée dans un récipient de dissolution (3) dans lequel l'iode est dissous en pentafluorure d'iode.

**3.** Procédé selon la revendication 1 ou 2, **caractérisé en ce qu'**on alimente en continu le pentafluorure d'iode d'un récipient de stockage (6) avec une pompe (2) en fonction de la consommation dans la boucle, qui conduit via le récipient de dissolution d'iode (3) au réacteur (1).

Fig.

CF₃CF₂I

TFE